(19) Europäisches Patentamt
European Patent Office
Office européen des brevets

(11) **EP 1 090 298 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**24.06.2015 Patentblatt 2015/26**

(45) Hinweis auf die Patenterteilung:
**19.07.2006 Patentblatt 2006/29**

(21) Anmeldenummer: 99931132.7

(22) Anmeldetag: **22.06.1999**

(51) Int Cl.:
*G01N 33/543* (2006.01)     *G01N 33/58* (2006.01)
*G01N 33/569* (2006.01)     *G01N 33/576* (2006.01)
*G01N 33/545* (2006.01)

(86) Internationale Anmeldenummer:
**PCT/EP1999/004310**

(87) Internationale Veröffentlichungsnummer:
**WO 1999/067643 (29.12.1999 Gazette 1999/52)**

(54) **VERBESSERUNG VON BINDEASSAYS DURCH MULTIEPITOPANALYSE**

IMPROVED BINDING ASSAYS THROUGH MULTIEPITOPE ANALYSIS

ESSAIS DE LIAISON AMELIORES PAR ANALYSE D'EPITOPES MULTIPLES

(84) Benannte Vertragsstaaten:
**AT CH DE ES FR GB IT LI NL**

(30) Priorität: **22.06.1998 DE 19827714**
**26.08.1998 DE 19838802**

(43) Veröffentlichungstag der Anmeldung:
**11.04.2001 Patentblatt 2001/15**

(60) Teilanmeldung:
**05008770.9 / 1 568 996**

(73) Patentinhaber: **Roche Diagnostics GmbH**
**68305 Mannheim (DE)**

(72) Erfinder:
• **KARL, Johann**
**D-82380 Peissenberg (DE)**
• **HORNAUER, Hans**
**D-82380 Peissenberg (DE)**

(74) Vertreter: **Weickmann & Weickmann**
**Postfach 860 820**
**81635 München (DE)**

(56) Entgegenhaltungen:
EP-A- 0 461 462     EP-A1- 0 139 373
EP-A1- 0 200 381     EP-A2- 0 171 150
WO-A-93/08472     WO-A-97/32212
WO-A1/94/24560     WO-A1-99/05525
US-A- 5 120 662     US-A- 5 627 026
US-B2- 6 815 217

• R. EKINS ET AL.: "Multianalyte microspot imunoassay. The microanalytical "compact disk" of the future." ANNALES DE BIOLOGIE CLINIQUE, Bd. 50, Nr. 5, 1992, Seiten 337-353, XP000617908 PARIS, FR.
• R. EKINS ET AL.: "Development of microspot multi-analyte ratiometric immunoassay using dual fluorescent--labelled antibodies." ANALYTICA CHIMICA ACTA, Bd. 227, Nr. 1, 1. Dezember 1989 (1989-12-01), Seiten 73-96, XP002108690 AMSTERDAM, NL ISSN: 0003-2670
• S. E. KAKABAKOS ET AL.: "Multianalyte immunoassay based on spatially distinct fluorescent areas quantified by laser-excited solid-phase time-resolved fluorometry " CLINICAL CHEMISTRY., Bd. 38, Nr. 3, März 1992 (1992-03), Seiten 338-342, XP002108659 AMERICAN ASSOCIATION FOR CLINICAL CHEMISTRY. WINSTON., US ISSN: 0009-9147
• R. DOUGLAS HOSTLER ET AL.: 'TANDEM® ICON®Strep A: A Highly Sensitive Rapid Test for Group A Streptococcal Infection Employing Particle Entrapment and ImmunoConcentration TM' ADVANCES IN INFECTIONS DISEASE DIAGNOSTICS 1987, Seiten 1 - 4

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zum Nachweis eines oder mehrerer Analyten in einer Probe, wobei der Nachweis des Analyten mit unterschiedlichen, an den Analyten bindefähigen Reagenzien geführt wird. Weiterhin umfaßt die Erfindung eine Festphase zum Nachweis eines Analyten, wobei die Festphase einen nichtporösen Träger und räumlich getrennte Testflächen umfaßt, wobei jede Testfläche unterschiedliche Reagenzien enthält.

Eine Vielzahl von Analyten kann durch immunologische Nachweisverfahren bestimmt werden. Solche immunologische Nachweisverfahren nutzen die spezifische Bindefähigkeit von Analyten mit bestimmten Reagenzien, wie beispielsweise Antigen-Antikörper-Wechselwirkungen. Grundsätzlich können immunologische Bestimmungen in einer Reihe von Testformaten durchgeführt werden, wie etwa dem Sandwichtestformat, dem indirekten Testformat, dem Rücktitrationsformat oder dem Brückenformat.

Ein zuverlässiger Nachweis von Infektionserkrankungen, z.B. einer Infizierung mit Viren wie etwa HIV, HBV oder HCV, ist von besonderem Interesse, um die Erkrankung bei betroffenen Personen möglichst frühzeitig diagnostizieren zu können. Im Allgemeinen werden immunologische Bestimmungen von Antikörpern gegen HIV, HBV oder HCV im indirekten Testformat oder mittels Brückenformat durchgeführt. Zum Nachweis der Antikörper werden dabei zumeist Gemische verschiedener Proteine bzw. Peptide verwendet, die Epitope aus der Core- und Envelope-Region des Erregers umfassen. Dieses Gemisch wird auf einem Träger, d.h. einer Festphase, immobilisiert. Da die Einstufung als HIV-positiv für den Einzelnen von großer Bedeutung ist und falsch positive Ergebnisse fatale Folgen haben können, ist es derzeit notwendig, alle bei dieser immunologischen Bestimmung in Routinetests erhaltenen positiven Ergebnisse in einem Bestätigungstest zu überprüfen. Als Bestätigungstest wird üblicherweise ein Westernblot verwendet, bei dem die einzelnen Proteinbestandteile eines Viruslysats auf einen porösen Träger aufgeblottet sind. Im Fall von HCV ist es allerdings sehr schwierig, den Virus zu züchten. Deshalb wird hier als Bestätigungstest kein Westernblot mit Viruslysat sondern ein RIBA (Recombinant Immunoblot Assay) durchgeführt, bei dem es sich um einen Immunodotblot, der rekombinante Proteine bzw. Peptide als Testreagenzien umfaßt, handelt.

Ein großer Nachteil der derzeit verwendeten Routinetests besteht darin, daß je nach Analyt Gemische von 5 bis 10 oder mehr Antigenen zum Nachweis eingesetzt werden. Zwar werden die Routinetests laufend verbessert, ein vollständiger Verzicht auf Bestätigungstests konnte jedoch noch nicht erreicht werden. Beispielsweise wird im Enzymun® HIV-Test (Boehringer Mannheim) ein Gemisch aus ca. 5 verschiedenen Antigenen verwendet, welche für den Nachweis sowohl biotinyliert als auch Digoxigenin-markiert sind. Obwohl der Test gut funktioniert, bedeutet die Verwendung von Antigengemischen mit einer derart hohen Anzahl verschiedener Antigene, daß die einzelnen auf der Festphase immobilisierten bzw. gebundenen Antigene nicht mehr in einer für den Nachweis optimalen Konzentration vorliegen können. Die Bindekapazität der Festphase ist bei einem solchen Gemisch aus einer Vielzahl von Komponenten nicht mehr ausreichend, um alle Antigene in der optimalen Konzentration zu binden. Weiterhin hat die Verwendung eines Antigen-Gemisches bei der Beschichtung einer Testfläche zur Folge, daß die verschiedenen Antigene um die Bindungsstellen auf der Festphase kompetieren und die unterschiedlichen Größenverhältnisse zu unterschiedlichen Diffusionsgeschwindigkeiten und zu unterschiedlichen sterischen Effekten führen. Bei einer Direktbeschichtung werden z.B. hydrophobe Antigene bevorzugt an die Kunststoffoberfläche gebunden, während gleichzeitig hydrophilere Antigene verdrängt werden. Dies führt dazu, daß zum einen nur schlecht reproduzierbare Ergebnisse erhalten werden und zum anderen die Konzentration bestimmter Antigen-Epitope so gering wird, daß ein signifikanter Nachweis nicht mehr möglich ist.

Ein weiterer Nachteil der Verwendung von Antigengemischen in Routinetests liegt darin, daß durch das Gemisch unterschiedlicher Antigene die Gefahr einer erhöhten unspezifischen Bindung deutlich gesteigert wird, was wiederum zu einem Anstieg falsch positiver Ergebnisse führt. Dies bewirkt, daß bei den bisher verwendeten Routinetests die Cut-off-Grenze relativ hoch gesetzt werden muß und somit Sensitivität verloren geht. Vor allem im Westernblot nimmt aufgrund von im Viruslysat vorliegenden Fremdproteinen die Zahl der falschpositiven Ergebnisse aufgrund unspezifischer Bindung deutlich zu, so daß mindestens 2 reaktive Banden für ein positives Ergebnis gefordert werden.

Es wurde versucht, die Sensitivität dieser Nachweisverfahren weiter zu verbessern. EP 0 461 462 A1 beschreibt einen Immunoassay zum Nachweis von viralen Antikörpern mit Hilfe eines indirekten Testkonzepts. Bei dem in EP 0 461 462 A1 beschriebenen Immunodotblot werden anstelle eines üblichen Viruslysats aufgereinigte rekombinante Proteine einzeln in diskreten Testflächen auf einen porösen Träger aufgetragen, wobei ein Testformat erhalten wird, das aufgrund der Verwendung aufgereinigter Proteine sensitiver als ein Westernblot ist.

EP 0 627 625 A1 betrifft ein Verfahren zum Nachweis von viralen Antikörpern in einer Probe mittels Brückenkonzept. Auch bei diesem Verfahren handelt es sich um einen RIBA (Recombinant Immunoblot Assay), bei dem mehrere Antigene räumlich getrennt auf eine Festphase aus einem porösen Material aufgebracht werden, wobei auf die Notwendigkeit der Verwendung einer Festphase aus porösem Material hingewiesen wird.

EP 0 445 423 A2 betrifft ein Verfahren zum Nachweis von HCV-Antikörpern mit Hilfe mehrerer Epitope eines HCV-Antigens. Auch in EP 0 445 423 A2 wird ein Immunodotassay zur Antikörperbestimmung beschrieben, wobei eine höhere Sensitivität durch die Verwendung bestimmter, verbesserter Antigene erzielt wird.

Ekins R. et al. (Ann. Biol. Clin., 1992, Vol. 50, S. 337 - 353) beschreibt einen Multianalyten-Mikrospot-Immunoassay, in dem ein theoretischer Ansatz zur Erhöhung der Sensitivität von Mikrospot-Assays zur Analyse mehrerer Analyten gegeben wird.

Ekins R. et al. (Analytica Chimica Acta, 1989, Vol. 227, S. 73- 96) beschreibt theoretische Überlegungen zu Multianalyten-Mikrospot-Immunoassays, mit denen es möglich sein sollte, im Prinzip $10^6$ verschiedene Substanzen in einem Probevolumen von 100 µl nachzuweisen.

Kakabakos et al. (Clin. Chem., 1992, Vol. 38/3, S. 338 - 342) betrifft einen Multianalyten-Immunoassay, bei dem die vier Analyten LH, FSH, TSH und PRL gleichzeitig quantitativ nachgewiesen werden.

WO 97/32212 offenbart ein optisches System zum Nachweis von mehreren Analyten unter Verwendung eines "Waveguides".

WO 93/08472 beschreibt das Konzept eines "Ambient-Analyt-Immunoassays", also eines vom eingesetzten Probevolumen unabhängigen Assays.

Bei diesen im Stand der Technik beschriebenen Verfahren ist jedoch aufgrund der Verwendung eines porösen Trägers das definierte Aufbringen einer vorbestimmten Reagenzmenge schwierig. Insbesondere besteht die Gefahr des Ineinanderlaufens der einzelnen aufgebrachten Testspots. Diese Nachteile werden um so gravierender, je kleiner die aufgebrachten Spots werden, so daß diese Verfahren insbesondere für miniaturisierte Testsysteme nicht geeignet sind. Außerdem ist die Handhabung von Papierstreifen schwer automatisierbar und somit als Routinetest nicht denkbar.

Eine Aufgabe der Erfindung bestand deshalb darin, ein Verfahren bereitzustellen, durch das die im Stand der Technik auftretenden Nachteile zumindest teilweise beseitigt werden können.

Diese Aufgabe wird erfindungsgemäß gelöst durch ein Verfahren zum Nachweis eines Analyten in einer Probe, umfassend die Schritte:

(a) Bereitstellen einer Festphase, die einen nichtporösen Träger und mindestens zwei räumlich getrennte Testflächen umfaßt, wobei die Testflächen jeweils unterschiedliche, immobilisierte analytspezifische Rezeptoren enthalten,
(b) Inkontaktbringen der Probe mit der Festphase und mindestens einem freien analytspezifischen Rezeptor, der eine signalgebende Gruppe trägt oder mit einer signalgebenden Gruppe bindefähig ist, und
(c) Nachweisen des Vorhandenseins oder/und der Menge des Analyten durch Bestimmung der signalgebenden Gruppe auf den Testflächen,

wobei der Analyt eine heterogene Antikörperpopulation oder ein heterogenes Antigengemisch ist, wobei die Antigene oder Antikörper von einem Erreger stammen bzw. induziert werden.

Der immobilisierte analytspezifische Rezeptor kann sowohl direkt als auch indirekt über einen oder mehrere Rezeptoren an die Festphase gebunden sein. Die Bindung kann beispielsweise durch adsorptive oder kovalente Wechselwirkungen, bevorzugt jedoch durch spezifische hochaffine Wechselwirkungen, z.B. Streptavidin oder Avidin/Biotin oder Antikörper-Antigen-Wechselwirkungen erfolgen.

Der freie analytspezifische Rezeptor kann selbst eine signalgebende Gruppe tragen oder mit einer signalgebenden Gruppe bindefähig sein. In diesem Fall besteht das Nachweisreagenz aus mehreren Komponenten.

Die einzelnen Testflächen binden im Fall von heterogenen Analytpopulationen eine Teilpopulation des zu bestimmenden Analyten. Die jeweils auf einer Testfläche immobilisierten analytspezifischen Rezeptoren sind unterschiedlich, d.h. sie binden erfindungsgemäß vorzugsweise an verschiedene Analytsubtypen wie etwa Antigensubtypen oder/und an verschiedene Analyttypen wie etwa unterschiedliche Antigene oder/und Antikörper.

Überraschenderweise wurde festgestellt, daß die Sensitivität von Nachweistests, wie etwa Antikörpertests, durch die Verwendung von Paneltests, bei denen die verschiedenen Reagenzien, z.B. verschiedene Antigene, als Einzelspots, d.h. einzeln auf separaten Testflächen, aufgetragen werden, deutlich verbessert werden kann. Durch die erfindungsgemäße Multiepitopanalyse, d.h. den gleichzeitigen separaten Nachweis mehrerer Teilpopulationen eines Analyten oder Erregers, wie etwa HIV, kann die Sensitivität und Zuverlässigkeit von Nachweistests erheblich gesteigert werden.

Wenn auf einer oder mehreren, in manchen Fällen auf mindestens zwei, Testflächen ein positives Testergebnis erhalten wird, wird dies als Vorhandensein des Analyten in der Probe gewertet.

Durch die erfindungsgemäße Verwendung eines nichtporösen Trägers, ist es möglich, die Reagenzien in definierten Flächen aufzubringen. Dies ist insbesondere bei miniaturisierten Testformaten von Bedeutung. Entsprechend weisen die Testflächen bevorzugt einen Durchmesser von 0,01 bis 1 mm, mehr bevorzugt von 0,1 bis 0,5 mm umd am meisten bevorzugt von 0,1 bis 0,2 mm auf.

Bevorzugt werden Festphasen mit mehreren Testflächen verwendet, die auch als Array-Systeme bezeichnet werden. Solche Array-Systeme sind z.B. bei Ekins und Chu (Clin. Chem. 37 (1995) 1955-1967) und in den US-Patenten 5,432,099, 5,516,635 und 5,126,276 beschrieben.

Die erfindungsgemäß verwendete Festphase umfaßt einen für Nachweisverfahren verwendbaren, nichtporösen Träger. Der nichtporöse Träger kann dabei aus einem beliebigen, nichtporösen Material bestehen. Bevorzugt umfaßt der Träger eine Kunststoff-, Glas-, Metall- oder Metalloxidoberfläche. Besonders bevorzugt weist der Träger eine Polystyroloberfläche auf. Auf diesem Träger sind räumlich diskrete Bereiche (Testflächen) angeordnet. Auf diesen Testflächen sind Reagenzien, wie etwa immobilisierte Festphasenrezeptoren aufgebracht. Die Immobilisierung der Reagenzien auf den Testflächen erfolgt nach bekannten Methoden, z.B. durch direkte adsorptive Bindung, durch kovalente Kopplung oder durch Kopplung über hochaffine Bindepaare, z.B. Streptavidin/Biotin, Antigen/Antikörper oder Zucker/Lectin.

Besonders vorteilhaft ist, wenn die räumlich getrennten Testflächen separat mit verschiedenen Reagenzien beladen werden. Durch die Einzelauftragung der verschiedenen Testflächen können für jedes Reagenz, beispielsweise für jedes einzelne Antigen, die optimale Festphasenkonzentration und die optimalen Beschichtungsbedingungen z.B. in Form von speziellen Pufferrezepturen gewählt werden. Dadurch ist es möglich, jeden einzelnen analytspezifischen Rezeptor, z.B. jedes einzelne Antigen bis zur maximalen Bindekapazität der Fläche zu beschichten, während bei den bisher bekannten Tests jeder Rezeptor, z. B. jedes Antigen nur zu einem Teil der verfügbaren Bindekapazität gebunden werden konnte. Durch die separate Auftragung der verschiedenen Reagenzien findet außerdem keine Kompetition der einzelnen Reagenzien, beispielsweise der Antigene, um die Bindungsplätze auf der Festphase statt. Entsprechend ist es bevorzugt, jeweils nur ein Reagenz, das spezifisch mit dem zu bestimmenden Analyten bindefähig ist, pro Testfläche zu binden, so daß jede Testfläche nur einen einzigen Typ eines immobilisierten analytspezifischen Rezeptors enthält. Dieses Reagenz kann gegebenenfalls durch inerte Verdünnermoleküle verdünnt werden, um eine optimale homogene Bindephase zu bilden. Inerte Verdünnermoleküle sind Moleküle, die an die Festphase binden aber keine Wechselwirkung mit dem Analyten oder anderen Probebestandteilen eingehen. Geeignete Verdünnermoleküle sind beispielsweise in WO 92/10757 und in EP 0 664 452 A2 beschrieben.

Bei Testflächen, auf denen nur ein einziges, mit dem Analyten bindefähiges Reagenz, wie etwa ein Antigen, gebunden ist, wurde festgestellt, daß die unspezifische Bindung deutlich reduziert ist. So ist z.B. bei Auftragung verschiedener Antigene als Einzelspots keine meßbare unspezifische Bindung zu beobachten, während ein Testspot, auf den ein Gemisch aus mehreren Antigenen aufgebracht wurde, eine deutlich meßbare unspezifische Bindung zeigt.

Der Nachweis des Analyten erfolgt im erfindungsgemäßen Verfahren auf bekannte Weise durch Verwendung geeigneter Markierungsgruppen, z.B. Fluoreszenzmarkierungsgruppen, chemilumineszierender Gruppen, radioaktiven Markierungen, Enzymmarkierungen, farbigen Markierungen und Solpartikeln. Alternativ kann, bei geeigneten Festphasen, die Wechselwirkung von Bestandteilen des Nachweismediums mit den Testflächen auch durch Bestimmung der Schichtdicke der jeweiligen Flächen z.B. durch Plasmonenresonanzspektroskopie, nachgewiesen werden.

Die begrenzten Testflächen können darüber hinaus zur Unterscheidung gegenüber inerten Bereichen der Festphase eine nachweisbare und analytunspezifische Markierungsgruppe enthalten, die neben der analytspezifischen Beschichtungsgruppe nachweisbar ist und mit ihr nicht interferiert. Ein Beispiel für eine solche analytunspezifische Markierungsgruppe ist eine Fluoreszenz-Markierungsgruppe, die bei einer Wellenlänge fluoresziert, die von der Fluoreszenzwellenlänge einer analytspezifischen Markierungsgruppe verschieden ist. Die analytunspezifische Markierungsgruppe wird vorzugsweise, ebenso wie der Festphasenrezeptor, über ein hochaffines Bindepaar, z.B. Streptavidin/Biotin immobilisiert.

Eine weitere Sensitivitätssteigerung kann durch die Verwendung eines universellen Nachweisreagenz erreicht werden. Zuvor kann für jedes Analytmolekül ein separates Nachweisreagenz, welches an das Analytmolekül bindet und eine Markierung, wie etwa ein Enzym, einen Fluoreszenzmarker oder fluoreszierende Latexpartikel trägt, eingesetzt werden. Durch die Kombination mehrerer markierter Nachweisreagenzien wird jedoch oftmals die Konzentration an Markierungen sehr hoch, so daß die unspezifische Bindung naturgemäß stark zunimmt. Dieses Problem kann durch die Verwendung eines universellen Nachweisreagenz gelöst werden. Erfindungsgemäß bevorzugt werden fluoreszenzmarkierte Latexpartikel als universelles Nachweisreagenz verwendet. Dabei wird für die spezifische Bindung des Analytmoleküls ein analytspezifischer erster Rezeptor verwendet, der selbt keine signalgebende Gruppe trägt. An diesen analytspezifischen ersten Rezeptor bindet ein universeller zweiter markierter Rezeptor, d.h. ein Rezeptor, der analytunabhängig an mehrere, vorzugsweise an alle verwendeten ersten Rezeptoren bindet. Die Kopplung des zweiten Rezeptors an die Markierungsgruppe kann adsorptiv, kovalent über funktionelle Gruppen erfolgen oder über hochaffine Bindepaare, z.B. Streptavidin/Biotin, Antigen/Antikörper oder Zucker/Lectin erfolgen. Bevorzugt wird das bekannte Dig/Anti-Dig-System verwendet.

Ein weiterer Nachteil der Brückentests, die z.B. als 1-Schritt-Reaktion ausgeführt werden, ist, daß der Festphasenrezeptor (z.B. biotinyliertes HIV-gp41) und der freie Nachweisrezeptor (z.B. digoxigenyliertes HIV-gp41) im Verhältnis 1:1 angeboten werden müssen, um ein optimales Signal zu erzielen. Dies ist nachteilig, da aufgrund der beschränkten Bindekapazität der Festphase die Konzentration der einzelnen Festphasenrezeptoren oftmals suboptimal ist und somit auch für den Nachweisrezeptor nicht günstig liegen kann.

Mit dem erfindungsgemäßen Verfahren können die Festphasenrezeptoren bereits auf der Festphase in optimaler Konzentration gebunden werden. Weiterhin kann auch der Nachweisrezeptor in optimaler Konzentration angeboten werden, da Rezeptorkonjugate mit Digoxigenin oder Biotin im Gegensatz zu Enzym-markierten Rezeptoren nur unwesentlich zu unspezifischer Bindung neigen. Man kann diese Reagenzien im Überschuß einsetzen, so daß Impräzisionen bei der Zugabe des Rezeptors nicht auf die Testpräzision durchschlagen.

Durch spezifische Bindung des zu bestimmenden Analyten an das auf die Testfläche immobilisierte Reagenz, z.B. einen Festphasenrezeptor, kann das Vorhandensein oder/und die Menge des Analyten in einer Probe bestimmt werden. Durch kombinierte Auswertung der verschiedenen Testflächen, die jeweils unterschiedliche, spezifisch mit dem Analyten bindefähige Reagenzien enthalten, kann die Sensitivität des Nachweisverfahrens, insbesondere durch eine Verringerung falsch positiver Ergebnisse und die zweifelsfreie Erkennung richtig positiver Ergebnisse, merklich verbessert werden. Von besonderem Interesse ist das erfindungsgemäße Verfahren zur Erfassung bzw. Eliminierung von unspezifischen Bindungen bei qualitativen Tests mit hohen Anforderungen an die Spezifität, wie etwa bei Tests auf Infektionen (z.B. HIV).

Durch die erfindungsgemäße Verwendung von Arrays, d.h. Festphasen, die mindestens zwei, mehr bevorzugt mindestens drei, am meisten bevorzugt mindestest fünf, und bis zu eintausend, mehr bevorzugt bis zu einhundert räumlich getrennte Testflächen umfassen, ist es möglich, mindestens eine dieser Testflächen so auszugestalten, daß sie eine Kontrollfläche darstellt. Folglich umfaßt das erfindungsgemäße Verfahren bevorzugt die Verwendung einer Festphase, die zusätzlich mindestens eine, mehr bevorzugt zwei und am meisten bevorzugt mindestens fünf Kontrollflächen umfaßt. Durch die Integration von Kontrollspots in die Festphase ist es möglich, falsche Resultate aufgrund von Störungen leicht und schnell zu erkennen. Neben den spezifischen Testflächen, ist es weiterhin möglich, einen probenspezifischen Untergrund zu messen und damit einen probenspezifischen Cut-off zu definieren. Die Verwendung eines Testarrays und die Verwendung von Kontrollspots ermöglicht es, die Cut-off-Grenze abzusenken. Der Cut-Off-Wert ist ein Grenzwert, der bei Testverfahren eingesetzt wird, um zwischen positiven und negativen Werten unterscheiden zu können. Ein solcher Cut-Off-Wert ist insbesondere bei Testverfahren, welche Infektionskrankheiten betreffen, von Bedeutung. Mit Hilfe des erfindungsgemäßen Verfahrens ist es möglich eine Positiv-/Negativ-Unterscheidung mit erheblich geringerer Fehlerwahrscheinlichkeit zu treffen.

Bei Verwendung von mehreren Testflächen, die jeweils zur Bestimmung unterschiedlicher Analytmoleküle vorgesehen sind, hat sich oftmals eine testflächenspezifische Definition des Cut-Off-Werts als geeignet erwiesen, um eine erhöhte Testspezifität (d.h. korrekte Unterscheidung zwischen positiven und negativen Werten) bei gleichbleibender Sensitivität zu erhalten.

Das erfindungsgemäße Verfahren kann für beliebigen Nachweismethoden eingesetzt werden, z.B. für Immunoassays, Nukleinsäure-Hybridisierungsassays, Zucker-Lectin-Assays und ähnliche Verfahren. Das erfindungsgemäße Verfahren eignet sich auch grundsätzlich zum Nachweis beliebiger Analyten in einer Probe. Besonders bevorzugt erfolgt der Nachweis des Analyten über spezifische Wechselwirkungen mit einem oder mehreren mit dem Analyten bindefähigen Reagenzien, d.h. Rezeptoren, die bevorzugt aus Proteinen, Peptiden, Antikörpern, Antigenen, Haptenen und Nukleinsäuren ausgewählt werden.

Während ein wesentlicher Vorteil des erfindungsgemäßen Verfahrens zunächst darin besteht, die Sensitivität des Nachweises eines einzigen Analyten zu verbessern, können bei geeigneter Wahl der Testflächen auch mehrere Analyten gleichzeitig mit hoher Sensitivität bestimmt werden.

Ein weiterer Gegenstand der vorliegenden Erfindung ist eine Festphase zum Nachweis eines Analyten in einer Probe, welche

**dadurch gekennzeichnet ist,**

dass sie einen nichtporösen Träger und mindestens zwei räumlich getrennte Testflächen umfasst, wobei die Testflächen jeweils unterschiedliche Reagenzien enthalten, die spezifisch den zu bestimmenden Analyten binden, wobei der Analyt eine heterogene Antikörperpopulation oder ein heterogenes Antigengemisch ist, wobei die Antigene und Antikörper von einem Erreger stammen oder induziert werden.

Bevorzugt enthalten die Testflächen jeweils unterschiedliche Reagenzien, die an verschiedene Epitope oder/und Subtypen eines Analyten oder/und an verschiedene Analyttypen binden.

Um eine möglichst große Anzahl von Testflächen auf einer Festphase unterzubringen, werden bevorzugt miniaturisierte Testformate verwendet. Der Abstand zwischen den einzelnen Testflächen wird so gewählt, daß ein Ineinanderlaufen der aufgebrachten Reagenzien nicht möglich ist. Üblicherweise reicht es hierfür aus, wenn die Ränder der Testflächen einen Abstand von 0,05 bis 5 mm aufweisen. Zwischen den Testflächen befindet sich bevorzugt eine inerte Oberfläche, die weder mit dem Analyten noch mit sonstigen Probebestandteilen bindefähig ist.

Die erfindungsgemäße Festphase kann in beliebigen Nachweisverfahren eingesetzt werden, z.B. in Immunoassays, Nukleinsäure-Hybridisierungsassays, Zucker-Lectin-Assays und dergleichen. Bevorzugt wird sie in einem Immunoassay zum Nachweis von Antikörpern oder/und Antigenen verwendet.

Weiterhin umfaßt die Erfindung ein Testkit zum Nachweis eines Analyten in einer Probe, welches eine erfindungsgemäße Festphase sowie markierte Nachweisreagenzien umfaßt. Markierte Nachweisreagenzien sind dem Fachmann bekannt und umfassen im allgemeinen eine Markierungsgruppe sowie eine spezifisch bindefähige Gruppe, die den Nachweis des Analyten ermöglicht. Geeignete Markierungsgruppen sind z.B. Fluoreszenz-, Chemilumineszenz-, Enzym-, radioaktive oder Partikel-(Sol)-Markierungsgruppen. Die spezifisch bindefähige Gruppe kann z.B. mit dem gebildeten Analytkomplex bindefähig sein oder bei kompetitiven Testformaten mit anderen Bestandteilen des Nachweissystems. Bevorzugt umfaßt der Testkit ein universelles Konjugat als Nachweisreagenz, insbesondere fluoreszenzmarkierte Latexpartikel, welches mit den für den Analyten spezifischen Nachweisrezeptoren bindefähig ist.

Ein weiteres Problem bei den bisher verfügbaren Routentests besteht darin, daß alle für einen Test, beispielsweise einen HIV-Test, notwendigen Antigene und Antikörper gemischt werden und für das Nachweisverfahren eine für dieses Gemisch optimale Cut-Off-Grenze festgelegt wird. Durch die Verwendung einer gemeinsamen Cut-Off-Grenze für alle Parameter wird die Cut-Off-Grenze jedoch durch die unspezifische Bindung des schlechtesten Einsatzstoffes bestimmt und begrenzt. Ein weiterer Gegenstand der vorliegenden Erfindung ist deshalb ein Verfahren zum Nachweis eines Analyten in einer Probe, umfassend die Schritte:

(a) Bereitstellen einer Festphase, die einen Träger und mindestens zwei räumlich getrennte Testflächen umfaßt, wobei die Testflächen jeweils unterschiedliche, immobilisierte analytspezifische Rezeptoren enthalten,
(b) Inkontaktbringen der Probe mit der Festphase und mindestens einem freien analytspezifischen Rezeptor, der eine signalgebende Gruppe trägt oder mit einer signalgebenden Gruppe bindefähig ist, und
(c) Nachweisen des Vorhandenseins oder/und der Menge des Analyten durch Bestimmung der signalgebenden Gruppe auf den Testflächen, wobei ein Signal oberhalb eines vorbestimmten Testflächenspezifischen Grenzwertes als positiv und unterhalb eines vorbestimmten Testflächen-spezifischen Grenzwertes als negativ klassifiziert wird.

Bei diesem Verfahren kann es sih bei dem Analyten um eine homogene oder um eine heterogene Population, z.B. eine heterogene Antikörperpopulation, ein Antigengemisch oder ein Gemisch von gegebenenfalls unterschiedlichen Antigenen und Antikörpern handeln, wobei die Antigene und Antikörper von einem oder mehreren Erregern stammen bzw. induziert werden. Die einzelnen Testflächen binden im Fall von heterogenen Analytpopulationen eine Teilpopulation des zu bestimmenden Analyten. Die jeweils auf einer Testfläche immobilisierten analytspezifischen Rezeptoren sind unterschiedlich, d.h. sie binden erfindungsgemäß vorzugsweise an verschiedene Epitope eines homogenen Analyten wie etwa eines Antigens, an verschiedene Analytsubtypen wie etwa Antigensubtypen oder/und an verschiedene Analyttypen wie etwa unterschiedliche Antigene oder/und Antikörper.

Durch die Verwendung von vorbestimmten Testflächeri-spezifischen Grenzwerten für jede einzelne Testfläche ist es möglich, die Spezifität von Nachweisverfahren deutlich zu verbessern, während die Sensitivität unverändert hoch bleibt. Der Grenzwert oder Cut-Off-Wert wird mittels der Größen Signal der Probe, Hintergrund der Probe und Hintergrund einer Negativkontrolle bestimmt. Eine übliche Berechnung des Cut-Off-Wertes (COI) erfolgt beispielsweise nach der Formel:

$$COI = Signal_{Probe} - Untergrund_{Probe} / n \times Untergrund_{Negativkontrolle}$$

Ein üblicher Wert für n beträgt beispielsweise 2. Der Faktor n - und damit der Cut-Off-Wert - kann für bestimmte Testflächen heraufgesetzt werden, bei denen falschpositive Proben beobachtet werden, wobei n eine Zahl zwischen 2 und 100, bevorzugt zwischen 2 und 10 sein kann.

Bevorzugt werden die Grenzwerte jeweils individuell für eine Testfläche bestimmt. Dies bedeutet, daß für die unterschiedlichen Testflächen unterschiedliche Grenzwerte bzw. Cut-Off-Werte festgelegt werden, insbesondere werden die Grenzwerte für mindestens zwei Testflächen unterschiedlich festgelegt. Bevorzugte Ausführungsformen dieses Verfahrens machen von den oben beschriebenen Merkmalen Gebrauch.

Die Erfindung wird durch die folgenden Beispiele weiter erläutert.

**Beispiele**

**1. Test auf Anti-HIV-Antikörper mit mehreren Antigen-spezifischen Testflächen mittels Microspot-Technologie**

Microspot ist eine miniaturisierte ultrasensitive Technologie, die ideal zur gleichzeitigen Bestimmung von verschiedenen Parametern in einem einzigen Meßvorgang geeignet ist. Im Fall der Bestimmung von Anti-HIV-Antikörpern werden verschiedene HIV-Nachweisantigene in sogenannten "Arrays" jeweils einzeln mittels einer Inkjet-Methode auf eine Testfläche (Spot) auf einem Polystyrolträger aufgebracht. Bei der Testdurchführung werden 30 $\mu$l mit Probenpuffer im Verhältnis 1:1 verdünnte Probe auf den mit Testflächen versehenen Träger pipettiert und 20 Minuten unter Schütteln bei Raumtemperatur inkubiert. Nach Absaugen der Probe und Waschen mit Waschpuffer werden 30 $\mu$l Reagenzlösung 1, die eine Mischung aller Digoxigenin-markierten HIV-Antigene enthält, zugegeben und wiederum 20 Minuten unter Schütteln bei Raumtemperatur inkubiert. Nach Absaugen der Reagenzlösung 1 und Waschen mit Waschpuffer werden 30 $\mu$l Reagenzlösung 2 mit Nachweisreagenz zugegeben. Als universelles Nachweisreagenz dienen 100 nm große, fluoreszierende Latexpartikel, die kovalent mit einem Anti-Digoxigenin-Antikörper beschichtet sind.

Dieses Na hweisreagenz wird wiederum 20 Minuten unter Schütteln bei Raumtemperatur inkubiert, anschließend abgesaugt, gewaschen und trockengesaugt. Die Testflächen werden dann mit einem He-Ne-Laser mit 633 nm Wellenlänge bestrahlt und die Fluoreszenz bei 670 nm Wellenlänge mit einer CCD-Kamera vermessen.

Die Festphase enthält spezifische Testflächen mit folgenden immobilisierten Antigenen:

- rekombinantes p24 Polypeptid
- rekombinante Reverse Transkriptase (RT)
- gp41-Peptid 1
- gp41-Peptid 2

Als Probenpuffer wurde ein 50 mM Tris-Puffer pH 7,6 mit folgenden Zusätzen verwendet: 0,05% Tween 20, 0,5% Rinderserumalbumin (RSA), 0,1% Rinder-IgG, 0,01% Methylisothiazolon, 3% Pepton.

Als Reagenzlösung 1 wurde der oben beschriebene Probenpuffer verwendet, der folgende testspezifische Antigene enthält:

- Digoxigenin-markiertes, rekombinantes p24
- Digoxigenin-markierte, rekombinante Reverse Transkriptase
- Digoxigenin-markiertes gp41-Peptid 1
- Digoxigenin markiertes pg41-Peptid 2

Als Reagenzlösung 2 wurde ein 50 mM Tris-Puffer pH 8,0 mit folgenden Zusätzen verwendet: 0,05% Tween 20, 0,9% NaCl, 0,5% RSA, 0,1 % Naazid und 0,01% von fluoreszenzmarkierten, einem monoklonalen Anti-Digoxigenin-Antikörper beschichteten Latexpartikein.

**2. Vergleich eines Anti-HIV-Antikörper-Tests im Microspot-Format zu konventionellen Methoden**

In diesem Versuch wurden sogenannte Serokonversionsproben vermessen. Diese Proben sind zeitlich aufeinanderfolgende Abnahmen verschiedener Personen, deren Serumbefund von HIV-negativ nach HIV-positiv konvertiert. Je sensitiver eine Testmethode ist, desto früher kann ein HIV-spezifisches Antikörper-Signal nachgewiesen werden. Die Proben wurden mit der erfindungsgemäßen Methode (Microspot) und vergleichend mit einer bekannten Methode (Enzymun® von Boehringer Mannheim) vermessen. Die dabei verwendeten, HIV-spezifischen Einsatzstoffe waren in beiden Testsystemen identisch, die sich daher vor allem nur durch die getrennte Einzelspot-Analyse unterscheiden. In der nachfolgenden Tabelle sind die Cut-off-Indices (Cut-off-Index = $\text{Signal}_{Probe}$-$\text{Signal}_{untergrund}$/2x$\text{Signal}_{Negativkontrolle}$) der beiden Methoden aufgetragen und zusätzlich mit Westernblot-Daten verglichen:

| Serokonversions Panel der Fa. BBI* | | Tag der Abnahme | p24 | RT | gp41-Peptid 1 | gp41-Peptid 2 | Western-Blot | Enzymun® |
|---|---|---|---|---|---|---|---|---|
| R | 2. Abnahme | 2 | 0.0 | 0.0 | 0.0 | 1.3 | negativ | 0.5 |
| | 3. Abnahme | 7 | 22.2 | 0.0 | 0.0 | 2.4 | indifferent | 15.4 |
| | 4. Abnahme | 13 | 17.4 | 2.6 | 4.4 | 36.4 | positiv | 36.0 |
| AB | 1. Abnahme | 0 | 0.0 | 0.0 | 0.6 | 0.0 | negativ | 0.3 |
| | 2. Abnahme | 28 | 0.0 | 0.0 | 0.4 | 1.1 | negativ | 0.7 |
| | 3. Abnahme | 33 | 0.0 | 0.0 | 5.5 | 54.4 | negativ | 24.2 |
| | 4. Abnahme | 35 | 0.8 | 0.2 | 6.0 | 33.2 | positiv | 26.7 |
| | 5. Abnahme | 37 | 15.2 | 5.1 | 4.6 | 33.0 | positiv | 28.9 |
| AD | 5. Abnahme | 21 | 0.0 | 0.0 | 0.0 | 0.0 | negativ | 0.3 |
| | 6. Abnahme | 25 | 0.2 | 0.0 | 1.6 | 3.7 | positiv | 0.9 |
| | 7. Abnahme | 28 | 14.1 | 0.3 | 11.1 | 65.5 | positiv | 24.5 |
| AG | 3. Abnahme | 13 | 0.0 | 0.0 | 0.0 | 0.0 | negativ | 0.4 |
| | 4. Abnahme | 27 | 0.0 | 0.0 | 0.0 | 1.1 | negativ | 0.6 |
| | 5. Abnahme | 34 | 6.0 | 5.3 | 0.0 | 106.9 | positiv | 8.1 |
| | 6. Abnahme | 50 | 6.1 | 5.4 | 0.0 | 65.4 | positiv | 3.1 |

(fortgesetzt)

| Serokonversions Panel der Fa. BBI* | | Tag der Abnahme | p24 | RT | gp41-Peptid 1 | gp41-Peptid 2 | Western-Blot | Enzymun® |
|---|---|---|---|---|---|---|---|---|
| | 7. Abnahme | 78 | 1.0 | 6.8 | 0.0 | 23.9 | positiv | 1.6 |
| | 8. Abnahme | 163 | 1.5 | 5.3 | 0.0 | 4.9 | positiv | 0.6 |
| | 9. Abnahme | 194 | 2.3 | 2.5 | 0.0 | 2.8 | positiv | 0.7 |
| AI | 1. Abnahme | 0 | 0.0 | 0.0 | 1.2 | 0.1 | indifferent | 0.8 |
| | 2. Abnahme | 7 | 0.6 | 0.5 | 54.7 | 44.9 | positiv | 30.2 |
| | 3. Abnahme | 11 | 1.1 | 0.7 | 18.8 | 22.5 | positiv | 30.2 |
| * Boston Biomedica Inc. | | | | | | | | |

Dieser Vergleich zeigt, daß durch die Aufteilung in Einzelspots mit jeweils optimalen Antigenkonzentrationen die Sensitivität im Vergleich zu bekannten Tests deutlich verbessert werden konnte. Von den 5 Serokonversions-Panels werden 7 Abnahmen früher positiv erkannt. Dies entspricht je nach Panel einer früheren Detektion der HIV-Infektion von 3 bis 7 Tagen. Auch im Vergleich zum Westernblot wurde eine deutliche Steigerung der Sensitivität mit 6 früher erkannten Abnahmen erreicht.

**3. Verbesserung der Testspezifität durch Testflächen-spezifische Cut-Off-Berechnung**

In bisher verfügbaren Routinetests werden alle für die Bestimmung notwendigen Antigene und Antikörper vermischt und eine für dieses Gemisch optimale Cut-Off-Grenze festgelegt. Diese wird durch die unspezifische Bindung des "schlechtesten" Einsatzstoffes bestimmt. Durch die erfindungsgemäße Microspot-Technologie kann man hingegen eine Testflächen-spezifische Cut-Off-Berechnung, die für jeden Einsatzstoff spezifisch ist, durchführen.
Bei identischer Berechnung des Cut-Off-Werts ($COI = Signal_{Probe}-Untergrund_{Probe}/2x\ Untergrund_{Negativkontrolle}$) der einzelnen Testflächen konnte mit dem HIV-Kombinationstest (Beispiel 3) in 1264 Proben folgende Spezifität erreicht werden:

- p24-Antigen: 100%
- Anti-HIV-Antikörper-Test: 99,52% (sechs falsch positive Bestimmungen)

Da die falsch positiven Bestimmungen ausschließlich in den beiden Testflächen für gp41 Peptid 2 und Reverse Transkriptase auftraten, wurden die Cut-Off-Grenzen für diese Testflächen auf folgende Grenzwerte heraufgesetzt:

$$gp41\text{-}Peptid\ 2: \quad COI = Signal_{Probe}\text{-}Untergrund_{Probe}/5\ x\ Untergrund_{Negativkontrolle}$$

$$RT: \quad COI = Signal_{Probe}\text{-}Untergrund_{Probe}/3\ x\ Untergrund_{Negativkontrolle}$$

Auf diese Weise konnte die Spezifität des HIV-Tests von 99,52% auf 99,92% (nur noch eine einzige falsch positive Bestimmung) verbessert werden. Auch die Sensitivtät des Tests blieb unbeeinflusst, da der Cut-Off-Index des sensitiven p24-Antigentests nicht verändert wurde. So kann bei unverändert hoher Sensitivität eine deutliche Verbesserung der Spezifität erzielt werden.

**Patentansprüche**

**1.** Verfahren zum Nachweis eines Analyten in einer Probe, umfassend die Schritte:

(a) Bereitstellen einer Festphase, die einen nicht porösen Träger und mindestens zwei räumlich getrennte Testflächen umfasst, wobei die Testflächen jeweils unterschiedliche, immobilisierte analytspezifische Rezeptoren enthalten,

(b) Inkontaktbringen der Probe mit der Festphase und mindestens einem freien analytspezifischen Rezeptor, der eine signalgebende Gruppe trägt oder mit einer signalgebenden Gruppe bindefähig ist, und

(c) Nachweisen des Vorhandenseins oder/und der Menge des Analyten durch Bestimmung der signalgebenden Gruppe auf den Testflächen, wobei der Analyt eine heterogene Antikörperpopulation oder ein heterogenes Antigengemisch ist, wobei die Antigene oder Antikörper von einem Erreger stammen bzw. induziert werden.

**2.** Verfahren nach Anspruch 1,
**dadurch gekennzeichnet,**
**dass** die Testflächen einen Durchmesser von 0,01 bis 1 mm aufweisen.

**3.** Verfahren nach einem der vorhergehenden Ansprüche,
**dadurch gekennzeichnet**
dass die Festphase durch separates, direktes spezifisches Aufbringen der unterschiedlichen analytspezifischen Rezeptoren auf die räumlich getrennten Testflächen hergestellt wird.

**4.** Verfahren nach einem der vorhergehenden Ansprüche,
**dadurch gekennzeichnet,**
dass die Beschichtung auf den Testflächen jeweils aus einem einzigen bindefähigen Molekültyp gebildet wird.

**5.** Verfahren nach einem der vorhergehenden Ansprüche,
**dadurch gekennzeichnet,**
**dass** man eine Festphase verwendet, die zusätzlich mindestens eine Kontrollfläche umfasst, die keinen analytspezifischen Rezeptor enthält.

**6.** Verfahren nach einem der vorhergehenden Ansprüche,
**dadurch gekennzeichnet,**
**dass** zum Nachweis aus dem Analyten und damit bindefähigen Reagenzien gebildeten Komplexen ein universelles Nachweisreagenz, insbesondere markierte Latexpartikel verwendet werden.

**7.** Festphase zum Nachweis eines Analyten in einer Probe,
**dadurch gekennzeichnet,**
**dass** sie einen nichtporösen Träger und mindestens zwei räumlich getrennte Testflächen umfasst, wobei die Testflächen jeweils unterschiedliche Reagenzien enthalten, die spezifisch den zu bestimmenden Analyten binden, wobei der Analyt eine heterogene Antikörperpopulation oder ein heterogenes Antigengemisch ist, wobei die Antigene und Antikörper von einem Erreger stammen oder induziert werden.

**8.** Verwendung einer Festphase nach Anspruch 7 in einem Immunoassay.

**9.** Testkit zum Nachweis eines Analyten in einer Probe umfassend eine Festphase nach Anspruch 7 sowie markierte Nachweisreagenzien.

**10.** Verfahren zum Nachweis eines Analyten in einer Probe, umfassend die Schritte:

(a) Bereitstellen einer Festphase, die einen Träger und mindestens zwei räumlich getrennte Testflächen umfasst, wobei die Testflächen jeweils unterschiedliche, immobilisierte analytspezifische Rezeptoren enthalten,

(b) Inkontaktbringen der Probe mit der Festphase und mindestens einem freien analytspezifischen Rezeptor, der eine signalgebende Gruppe trägt oder mit einer signalgebenden Gruppe bindefähig ist, und

(c) Nachweisen des Vorhandenseins oder/und der Menge des Analyten durch Bestimmung der signalgebenden Gruppe auf den Testflächen, wobei ein Signal oberhalb eines vorbestimmten testflächenspezifischen Grenzwerts als positiv und unterhalb eines vorbestimmten testflächenspezifischen Grenzwerts als negativ klassifiziert wird.

**11.** Verfahren nach Anspruch 10,
**dadurch gekennzeichnet,**
**dass** die Grenzwerte jeweils individuell für eine Testfläche bestimmt werden.

**Claims**

1. Method for detecting an analyte in a sample, comprising the steps of:

   a) providing a solid phase which comprises a non-porous carrier and at least two spatially separated test surfaces, wherein each of the test surfaces contains different, immobilised analyte-specific receptors,
   b) bringing the sample into contact with the solid phase and at least one free analyte-specific receptor which carries a signal-emitting group or is bindable with a signal-emitting group, and
   c) detecting the presence and/or the amount of the analyte by determining the signal-emitting group on the test surfaces, wherein the analyte is a heterogeneous antibody population or a heterogeneous antigen mixture, wherein the antigens or antibodies come from or are induced by a pathogen.

2. Method according to claim 1, **characterised in that** the test surfaces have a diameter of from 0.01 to 1 mm.

3. Method according to either of the proceeding claims, **characterised in that** the solid phase is produced by means of separate, direct specific application of the different analyte-specific receptors to the spatially separated test surfaces.

4. Method according to any of the preceding claims, **characterised in that** the coating on the test surfaces is formed, in each case, from a single bindable molecule type.

5. Method according to any of the preceding claims, **characterised in that** a solid phase is used which comprises, in addition, at least one control surface which does not contain any analyte-specific receptors.

6. Method according to any of the preceding claims, **characterised in that** a universal detection reagent, in particular marked latex particles, is used for detecting the complexes formed from the analyte and the reagents which are bindable therewith.

7. Solid phase for detecting an analyte in a sample, **characterised in that** it comprises a non-porous carrier and at least two spatially separated test surfaces, the test surfaces each containing different reagents which specifically bind the analytes to be determined, the analyte being a heterogeneous antibody population or a heterogeneous antigen mixture, the antigens and antibodies coming from or being induced by a pathogen.

8. Use of a solid phase according to claim 7 in an immunoassay.

9. Test kit for detecting an analyte in a sample comprising a solid phase according to claim 7 and marked detection reagents.

10. Method for detecting an analyte in a sample, comprising the steps of

    a) providing a solid phase which comprises a carrier and at least two spatially separated test surfaces, wherein each of the test surfaces contains different, immobilised analyte-specific receptors,
    b) bringing the sample into contact with the solid phase and at least one free analyte-specific receptor which carries a signal-emitting group or is bindable with a signal-emitting group, and
    c) detecting the presence and/or the amount of the analyte by determining the signal-emitting group on the test surfaces, wherein a signal above a predetermined test-specific threshold value is classified as positive and a signal below a predetermined test-specific threshold value is classified as negative.

11. Method according to claim 10, **characterised in that** the threshold values are determined individually for a test surface in each case.

**Revendications**

1. Procédé de détection d'un analyte dans un échantillon, comprenant les étapes de :

   (a) mise à disposition d'une phase solide qui comprend un support non poreux et au moins deux surfaces de test spatialement séparées, dans laquelle les surfaces de test contiennent respectivement des récepteurs immobilisés différents spécifiques d'un analyte,

(b) la mise en contact de l'échantillon avec la phase solide et au moins un récepteur libre spécifique d'un analyte, qui porte un groupe donneur de signal ou qui peut se lier à un groupe donneur de signal, et

(c) la détection de la présence et/ou de la, quantité d'analyte par détermination du groupe donneur de signal sur les surfaces de test, dans laquelle l'analyte est une population d'anticorps hétérogène ou un mélange antigénique hétérogène, dans lequel les antigènes ou les anticorps proviennent d'un agent pathogène ou sont induits par celui-ci.

**2.** Procédé selon la revendication 1,
**caractérisé en ce que**
les surfaces de test présentent un diamètre de 0,01 à 1 mm.

**3.** Procédé selon l'une des revendications précédentes,
**caractérisé en ce que**
la phase solide est produite par application séparée, spécifique directe des récepteurs différents spécifiques d'un analyte sur les surfaces de test spatialement séparées.

**4.** Procédé selon l'une des revendications précédentes,
**caractérisé en ce que**
le revêtement sur les surfaces de test est formé respectivement d'un type de molécule unique pouvant se lier.

**5.** Procédé selon l'une des revendications précédentes,
**caractérisé en ce que**
l'on utilise une phase solide qui comprend en outre au moins une surface de contrôle qui ne contient pas de récepteur spécifique d'un analyte.

**6.** Procédé selon l'une des revendications précédentes,
**caractérisé en ce que**
pour la détection des complexes formés de l'analyte et des réactifs pouvant ainsi s'y lier, on utilise un réactif de détection universel, en particulier des particules de latex marquées.

**7.** Phase solide pour la détection d'un analyte dans un échantillon,
**caractérisé en ce qu'**elle comprend
un support non poreux et au moins deux surfaces de test séparées spatialement, dans laquelle les surfaces de test contiennent respectivement des réactifs différents qui se lient spécifiquement aux analytes à déterminer, dans laquelle l'analyte est une population d'anticorps hétérogène ou un mélange d'antigènes hétérogène, dans laquelle les antigènes et les anticorps proviennent d'un agent pathogène ou sont induits par celui-ci.

**8.** Utilisation d'une phase solide selon la revendication 7, dans un immuno-essai.

**9.** Kit de test pour la détection d'un analyte dans un échantillon, comprenant une phase solide selon la revendication 7 et des réactifs de détection marqués.

**10.** Procédé pour la détection d'un analyte dans un échantillon, comprenant les étapes de :

(a) mise à disposition d'une phase solide qui comprend un support et au moins deux surfaces de test spatialement séparées, dans laquelle les surfaces de test contiennent respectivement des récepteurs immobilisés différents, spécifiques d'un analyte,

(b) la mise en contact de l'échantillon avec la phase solide et au moins un récepteur libre spécifique d'un analyte, qui porte un groupe donneur de signal ou qui peut se lier à un groupe donneur de signal, et

(c) la détection de la présence et/ou de la quantité d'analyte par détermination du groupe donneur de signal sur les surfaces de test, dans laquelle un signal supérieur à une valeur seuil prédéterminée spécifique des surfaces de test est classé comme étant positif et un signal inférieur à une valeur seuil prédéterminée spécifique des surfaces de test est classé comme étant négatif.

**11.** Procédé selon la revendication 10,
**caractérisé en ce que**
les valeurs seuils sont déterminées respectivement individuellement pour une surface de test.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0461462 A1
- EP 0627625 A1
- EP 0445423 A2
- WO 9732212 A
- WO 9308472 A
- US 5432099 A
- US 5516635 A
- US 5126276 A
- WO 9210757 A
- EP 0664452 A2

**In der Beschreibung aufgeführte Nicht-Patentliteratur**

- **EKINS R. et al.** *Ann. Biol. Clin.,* 1992, vol. 50, 337-353
- **EKINS R. et al.** *Analytica Chimica Acta,* 1989, vol. 227, 73-96
- **KAKABAKOS et al.** *Clin. Chem.,* 1992, vol. 38 (3), 338-342
- **EKINS ; CHU.** *Clin. Chem.,* 1995, vol. 37, 1955-1967